# EUROPEAN PATENT APPLICATION

(11) **EP 4 610 270 A1**
(43) Date of publication of application: **03.09.2025**
(21) Application number: 23884684.4
(22) Date of filing: 25.10.2023
(51) Int. Cl.: C07K 7/08, A61K 38/10, A61P 31/00

(54) **NOVEL ANTIMICROBIAL PEPTIDE AND PHARMACEUTICAL COMPOSITION THEREOF**

(30) Priority: 01.11.2022 CN 202211358930
(71) Applicant: Qingdao Sensheng Biopharmaceutical Technology Co., Ltd, Qingdao, Shandong 266555 (CN)
(72) Inventor: SU, Feng, Qingdao, Shandong 266555 (CN); XU, Qingliang, Qingdao, Shandong 266555 (CN); LIU, Su, Qingdao, Shandong 266555 (CN); ZHANG, Shicui, Qingdao, Shandong 266555 (CN)
(74) Representative: Plavsa, Olga
(86) International application number: PCT/CN2023/126448
(87) International publication number: WO 2024/093752

(57) **Abstract**

Provided are an antimicrobial peptide that is safe and has high stability, a good antimicrobial effect, and a shorter sequence, as well as pharmaceutical composition thereof, which can be used for treating and relieving rosacea, adolescent acne, atopic dermatitis, genital warts, vulval intraepithelial neoplasia, inflammatory responses, inflammatory response syndrome, etc. The antimicrobial polypeptide comprises the amino acid sequence Ac-SRMKKWAKIIEKWRKWH-NH₂.

## Description

### Technical Field

The present disclosure relates to the field of biopharmacy, and in particular, to an antimicrobial peptide and use thereof.

### Background

Antimicrobial peptides (AMPs) are immune active molecules produced by organisms during the long-term evolution process to adapt to the environment, have antimicrobial, antiviral, antifungal and antiparasitic functions, play an important role in the natural immune defense system of the body, and are also called "host defense peptides" (HDPs). The AMPs are typically composed of 12-50 amino acid residues, most of which are cationic short peptides, with the charge carrying amount of between +2 and +9, and the antimicrobial peptide with the charge of +2 to +4 is most abundant (Rodriguez AA, Otero-González A, Ghattas M, Ständker L. Discovery, optimization, and clinical application of natural antimicrobial peptides. Biomedicines, 2021, 9(10): 1381. Yount NY, Bayer AS, Xiong YQ, Yeaman MR. Advances in antimicrobial peptide immunobiology. Biopolymers, 2006, 84(5): 435-458.). The reason why the AMP surface carries a large amount of positive charge is that the initial interaction of the AMPs with the bacterial cell membrane is mainly achieved by the electrostatic interaction between the cationic residues on the surface of the AMPs and the anions on the bacterial cell membrane. In addition, there are also a small amount of anionic AMPs, but the antimicrobial activity of the anionic AMPs is much lower than that of the cationic AMPs, and the role of the anionic AMPs in innate immunity is not yet fully understood, so the research on the AMPs mainly focuses on the cationic AMPs (Teixeira V, Feio MJ, Bastos M. Role of lipids in the interaction of antimicrobial peptides with membranes. Progress in Lipid Research, 2012, 51(2): 149-177). Structurally, the AMPs have three configurations: an α-helix configuration, a β-fold configuration, and an extended/randomly coiled configuration, which are amphipathic or have the ability to become amphipathic, allowing the AMPs to dissolve in water and lipid-rich environments (Lazzaro BP, Zasloff M, Rolff J. Antimicrobial peptides: application informed by evolution. Science, 2020, 368(6490): eaau5480.), and thus adhere to bacterial cell membranes and serve for membrane lysis. The AMPs also have small molecular weights, typically less than 10 kDa, which facilitate their rapid diffusion and secretion in host cells, triggering immediate defense against pathogenic microorganisms. To date, the Antimicrobial Peptide Database (APD) contains more than 3000 AMPs, covering antibacterial, antifungal, antiviral, antiparasitic, and other functions.

In recent decades, the abuse of antibiotics has led to the problem of bacterial drug resistance becoming a public health crisis. According to the latest survey of the Center for Disease Control and Prevention (CDC), antibiotic resistance causes millions of diseases worldwide every year, and it is estimated that the number of deaths caused by antibiotic resistance will reach tens of millions by 2050. Compared with traditional antibiotics, the application advantages of the AMPs mainly include: (1) selective toxicity: the difference in cell membrane composition between pathogens and hosts is the basis for the targeting specificity of the AMPs, so that the AMPs can specifically invade pathogens (Zhang QY, Yan ZB, Meng YM, Hong XY, Shao G, Ma JJ, Cheng XR, Liu J, Kang J, Fu CY. Antimicrobial peptides: mechanism of action, activity and clinical potential. Military Medical Research, 2021, 8(1): 48.); (2) rapid action: the time required for the AMPs to kill target bacteria is much shorter than the doubling time of target bacteria (Brogden KA. Antimicrobial peptides: pore formers or metabolic inhibitors in bacteria? Nature Reviews Microbiology, 2005, 3(3): 238-250.); (3) effective killing of persister: persister is a subpopulation of bacteria that can survive in a lethal dose of antibiotics and other stress environments in the bacterial population, and is a bacterial cell that has not undergone genetic mutation, has low metabolic activity and will not be killed by antimicrobial drugs (Liu XL, Yang WX, Ma YL, Wang D, Chen H. Research progress in the formation mechanism and treatment of persister. Chinese Journal of Infection Control, 2020, 19(2): 184-188. (in Chinese)). Due to the fact that the target of the AMPs is usually on the cell membrane and does not depend on cellular metabolic activity, it is beneficial for killing persister (Defraine V, Schuermans J, Grymonprez B, Govers SK, Aertsen A, Fauvart M, Michiels J, Lavigne R, Briers Y. Efficacy of artilysin art-175 against resistant and persistent Acinetobacter baumannii. Antimicrobial Agents and Chemotherapy, 2016, 60(6): 3480-3488.); (4) broad spectrum: in addition to common Gram negative and Gram positive bacteria, the AMPs are also effective against viruses, protozoa, and fungi (Yeung ATY, Gellatly SL, Hancock REW. Multifunctional cationic host defence peptides and their clinical applications. Cellular and Molecular Life Sciences: CMLS, 2011, 68(13): 2161-2176.); and (5) Not prone to developing drug resistance: the AMPs act on highly conserved cell membranes and can hardly be modified by bacteria to develop drug resistance; and the AMPs have a short half-life and are difficult to accumulate in the environment to induce bacteria to develop drug resistance; in addition, the AMPs have a short duration of action, and compared with antibiotics, the intermediate therapeutic concentration range of the AMPs is smaller than that of antibiotics, the smaller the intermediate therapeutic concentration range, the less likely it is to induce drug resistance. At present, the AMPs have been applied in the fields of food, agriculture, medicine, etc. and have achieved good results. In addition, the AMPs have certain applications in clinical practice. For example, the synthetic analog Omiganan of indolicidin has great potential in the treatment of fungal infections and can be used to treat rosacea, adolescent acne, atopic dermatitis, genital warts, and vulvar intraepithelial neoplasia (Zyrek D, Wajda A, Czechowicz P, Nowicka J, Jaśkiewicz M, Neubauer D, Kamysz W. The antimicrobial activity of omiganan alone and in combination against Candida isolated from vulvovaginal candidiasis and bloodstream infections. Antibiotics: Basel, Switzerland, 2021, 10(8): 1001.).

Although the AMPs have a bright future, there are many problems, mainly in three aspects. (1) Pharmacokinetics: due to the problems of hemolysis, low oral bioavailability, sensitivity to gastrointestinal protease degradation and the like, most of the AMPs cannot be administered orally; however, through systemic administration such as intravenous injection, due to the rapid degradation of the AMPs by proteolytic enzymes in plasma, the rapid clearance of the AMPs by the liver and kidneys of the body results in a short half-life of the AMPs, and therefore, the AMPs are limited to local topical administration only. (2) Antimicrobial activity: although the AMPs have a broad-spectrum antimicrobial effect, compared with traditional antibiotics, the antimicrobial activity of the AMPs is relatively low. That is, to achieve the same antimicrobial effect, the concentration of the AMPs may be higher (Chen X, Zhang M, Zhou CH, Kallenbach NR, Ren DC. Control of bacterial persister cells by Trp/Arg-containing antimicrobial peptides. Applied and Environmental Microbiology, 2011, 77(14): 4878-4885.), and high concentration of the AMPs will increase the hemolysis of host red blood cells. (3) Drug production and processing: compared with traditional small molecule therapeutic drugs, the AMPs have longer sequences and higher production costs, especially for disulfide-rich AMPs, which to some extent limit the production and use of the AMPs clinically.

At present, many strategies have been proposed to solve the problems of the AMPs, such as chemical modification of the AMPs to improve stability, substitution of amino acid sequences of the AMPs to improve antimicrobial activity, endocytic fusion of the AMPs to reduce cytotoxicity, and design of short linear AMPs to reduce cost. But based on the above optimization strategies of the AMPs, the improvement effect cannot achieve good results at the same time, and an artificial intelligence development method is proposed in the later stage, that is, to establish a prediction model to estimate the molecular properties of the AMPs for candidate screening, usually using manually selected or automatically learned components, structures, and physicochemical feature sets to build a prediction model; the candidate AMPs are usually obtained by combining and counting reasonable subsequences, and then randomly selecting or modifying from the existing molecular library. Moreover, by representing the amino acid residues of the AMPs in the form of a string, the amino acid sequence dataset of the AMPs is trained and analyzed by machine learning/deep learning, so as to efficiently identify amino acid sequences of novel AMPs.

Moreover, many researchers have developed strategies to design antimicrobial agents based on natural template peptides by integrating the knowledge of microorganisms and protein polypeptides. Compared with natural AMPs, the modified and designed AMPs exhibit more excellent antimicrobial effects and biocompatibility. Specifically, firstly, based on the previous summary of the nature of natural AMPs, the ratio of positive charge to hydrophobic amino acids is increased to improve the antimicrobial activity. Secondly, the secondary structure of the polypeptide is adjusted. Most AMPs have disordered structures in aqueous solution, but they interact with phospholipid biomolecules to form highly stable secondary structures after entering the membrane environment, which is important for antimicrobial activity. Therefore, the secondary structure is the essential element for the interaction between the AMP and membrane, and folding into a regular secondary structure helps AMP insert into the bacterial membrane. Thirdly, the regulation may be made to AMP amphipathicity. Amphipathicity refers to the degree of spatial separation of hydrophobic and hydrophilic residues on either side of the molecular backbone. Typically, amphipathicity is quantified by the hydrophobic moment, which is the vector sum of individual hydrophobic amino acids in a normalized helix. The amphipathic α-helix is the most common conformation, in which the main chain of the peptide chain spirals up as a regular helix around the central axis, with each 3-4 amino acid residues spiraling once. The hydrophilic surface of the amphipathic α-helical peptide and the negatively charged components of the bacterial membrane exert an electrostatic effect, and the hydrophobic surface of the peptide is inserted into the membrane, thereby increasing the permeability of the membrane. The AMPs can be usually modified by deleting, substituting, or adding amino acids to the sequence to form a perfect amphipathic structure. However, it was found that although AMPs with a perfect amphipathic structure have the best activity, their cytotoxicity also increases, indicating that this perfect amphipathic structure non-selectively increases membrane damage.

Although various polypeptide design optimization techniques have achieved some success, AMPs themselves still have some shortcomings, such as high toxicity, unclear toxicology, and immature clinical trial stability, which have become the biggest obstacle to application. In addition, most previous studies have only focused on simply cutting or modifying natural AMPs to obtain stronger performance, but often ignore the inherent structure and performance of the peptide sequence itself, resulting in the inability to fundamentally solve the poor performance of most AMPs, and few antimicrobial templates are suitable for practical application.

In previous studies, the amino and carboxyl terminals of phosvitin (Pv) from zebrafish were cut off respectively, and it was found that the 194 amino acids at the carboxyl terminal still had antimicrobial activity, while the 55 amino acid sequence (Pt6) at the amino terminal completely lost antimicrobial activity. The amino acids at the carboxyl terminal were shortened in proportion, and it was finally found that the 55 amino acids at the carboxyl terminal still had antimicrobial activity, and was named phosvitin-derived peptide (Pt5) (Wang SHWang YMa J, Ding YCZhang SC: Phosvitin Plays a Critical Role in the Immunity of Zebrafish Embryos via Acting as a Pattern Recognition Receptor and an Antimicrobial Effector Journal of Biological Chemistry 2011286(25):22653-22664). Since the phosvitin-derived peptide (Pt5) is derived from phosvitin (Pv) in vitellogenin (Vg), most of the antimicrobial proteins have amphiphilic molecular characteristics, generally a net positive charge of 2 to 9, and a high proportion of hydrophobic amino acids (Manchekar MRichardson PE, Forte TM.Datta GSegrest JPDashti N:Apolipoproteinbcontaining lipoprotein particle assembly-Lipid capacity of the nascent lipoprotein particle Journal of Biological Chemistry 2004279(38):39757-39766). Subsequent studies found that Pt5 not only has antimicrobial function, but also has certain protective effect on zebrafish infected by Aeromonas hydrophila (Ding YCLiu XMBu LZLi HYZhang SC: Antimicrobial-immunomodulatory activities Of zebrafish phosvitin-derived peptide Pt5.Peptides 201237(2):309-313.).

In conclusion, natural AMPs have a series of problems such as weak biological activity, high toxicity and low stability, which are caused by the limitation of their own mechanism, so most AMPs cannot be directly applied in practical production. From the existing technology and previous research, it has been found that the present disclosure screens and detects active sites of the AMPs, and then modifies and reconstructs the AMPs using the active site sequence as a template, creatively solving the practical application problems of the AMPs.

### Summary

The present disclosure removes the carboxyl terminal and amino terminal of vitellogenin Pv respectively by using the amino acid truncation expression method, obtains an antimicrobial polypeptide with the active site, designs and reconstructs the antimicrobial polypeptide by using the active site sequence as a template, and finally screens to obtain the antimicrobial polypeptide with high stability, good safety, good antimicrobial effect and shorter sequence. Based on this, the following invention has been completed.

In a first aspect, the present disclosure provides a novel antimicrobial polypeptide, comprising the amino acid sequence Ac-SRMKKWAKIIEKWRKWH-NH₂.

Further, the amino acid sequence Ac-SRMKKWAKIIEKWRKWH-NH₂ may have substitution of one and/or more amino acids, and the antimicrobial activity of the substituted amino acid sequence is not significantly reduced, or remains the same, or is significantly enhanced. Still further, 1-5 amino acids, preferably 1-3 amino acids, and more preferably 1 amino acid, are substituted. The substituted amino acids may be substituted continuously and/or at interval.

Further, the amino acid sequence Ac-SRMKKWAKIIEKWRKWH-NH₂ has one and/or more amino acids added to the carboxyl terminal and/or amino terminal, and the antimicrobial activity of the extended amino acid sequence is not significantly reduced, or remains the same, or is significantly enhanced. Still further, 1-30 amino acids, preferably 1-20 amino acids, and more preferably 1-10 amino acids, are added. The added amino acids may be concentrated at the carboxyl terminal, or at the amino terminal, or at both the carboxyl and amino terminals.

Further, the amino acid sequence Ac-SRMKKWAKIIEKWRKWH-NH₂ has 1-5 amino acids added or deleted, preferably 1-3 amino acids added and/or deleted, and further preferably 1 amino acid added and/or deleted, and the added or deleted amino acids may be concentrated or dispersed at any position of the amino acid sequence, and the antimicrobial activity of the altered amino acid sequence is not significantly reduced, or remains the same, or is significantly enhanced.

Further, the spatial configuration of the amino acid sequence Ac-SRMKKWAKIIEKWRKWH-NH₂ or a mutant sequence thereof (the amino acid sequence after amino acid substitution, addition, or deletion as described above) is an α-helix configuration, a β-fold configuration, and/or an extended/randomly coiled configuration.

In a second aspect, the present disclosure provides an antimicrobial polypeptide comprises an amino acid sequence having 90-100% homology with the amino acid sequence Ac-SRMKKWAKIIEKWRKWH-NH₂, which is referred to as the homologous sequence of Ac-SRMKKWAKIIEKWRKWH-NH₂, and the spatial configuration of the homologous sequence is an α-helix configuration, a β-fold configuration, and/or an extended/randomly coiled configuration.

In a third aspect, the present disclosure provides an antimicrobial pharmaceutical composition, containing the antimicrobial polypeptide of the first aspect of the present disclosure and/or the antimicrobial polypeptide of the second aspect of the present disclosure, as well as a pharmaceutically acceptable excipient.

In a specific embodiment, the antimicrobial pharmaceutical composition of the present disclosure only contains the antimicrobial polypeptide of the first aspect of the present disclosure as an active ingredient, wherein the antimicrobial polypeptide comprises the amino acid sequence Ac-SRMKKWAKIIEKWRKWH-NH₂.

Further, the amino acid sequence Ac-SRMKKWAKIIEKWRKWH-NH₂ may have substitution of one and/or more amino acids, and the antimicrobial activity of the substituted amino acid sequence is not significantly reduced, or remains the same, or is significantly enhanced. Still further, 1-5 amino acids, preferably 1-3 amino acids, and more preferably 1 amino acid, are substituted. The substituted amino acids may be substituted continuously and/or at interval.

Further, the amino acid sequence Ac-SRMKKWAKIIEKWRKWH-NH₂ has one and/or more amino acids added to the carboxyl terminal and/or amino terminal, and the antimicrobial activity of the extended amino acid sequence is not significantly reduced, or remains the same, or is significantly enhanced. Still further, 1-30 amino acids, preferably 1-20 amino acids, and more preferably 1-10 amino acids, are added. The added amino acids may be concentrated at the carboxyl terminal, or at the amino terminal, or at both the carboxyl and amino terminals.

Further, the amino acid sequence Ac-SRMKKWAKIIEKWRKWH-NH₂ has 1-5 amino acids added or deleted, preferably 1-3 amino acids added and/or deleted, and further preferably 1 amino acid added and/or deleted, and the added or deleted amino acids may be concentrated or dispersed at any position of the amino acid sequence, and the antimicrobial activity of the altered amino acid sequence is not significantly reduced, or remains the same, or is significantly enhanced.

Further, the spatial configuration of the amino acid sequence Ac-SRMKKWAKIIEKWRKWH-NH₂ or a mutant sequence thereof (the amino acid sequence after amino acid substitution, addition, or deletion as described above) is an α-helix configuration, a β-fold configuration, and/or an extended/randomly coiled configuration.

In another specific embodiment, the antimicrobial pharmaceutical composition of the present disclosure only contains the antibacterial polypeptide of the second aspect of the present disclosure as an active ingredient, wherein the antimicrobial polypeptide comprises an amino acid sequence having 90-100% homology with the amino acid sequence Ac-SRMKKWAKIIEKWRKWH-NH₂, referred to as the homologous sequence of Ac-SRMKKWAKIIEKWRKWH-NH₂, and the spatial configuration of the homologous sequence is an α-helix configuration, a β-fold configuration, and/or an extended/randomly coiled configuration.

In another specific embodiment, the antimicrobial pharmaceutical composition of the present disclosure contains both the antimicrobial polypeptide according to the first aspect of the present disclosure and the antimicrobial polypeptide according to the second aspect of the present disclosure as active ingredients.

In another specific embodiment, the antimicrobial pharmaceutical composition of the present disclosure further contains other active ingredients different from the antimicrobial polypeptide of the first aspect and the second aspect of the present disclosure, wherein the other active ingredients may be antibiotics, immune modulators, minerals, trace elements, and/or vitamins.

Further, the antimicrobial pharmaceutical composition of the present disclosure can be prepared in dosage forms such as injections, oral preparations, lyophilized powder for injection, etc.

In a fourth aspect, the present disclosure provides a nucleic acid molecule, wherein the nucleic acid molecule encodes an antimicrobial polypeptide, and the antimicrobial polypeptide comprises the amino acid sequence Ac-SRMKKWAKIIEKWRKWH-NH₂.

Further, the amino acid sequence Ac-SRMKKWAKIIEKWRKWH-NH₂ may have substitution of one and/or more amino acids, and the antimicrobial activity of the substituted amino acid sequence is not significantly reduced, or remains the same, or is significantly enhanced. Still further, 1-5 amino acids, preferably 1-3 amino acids, and more preferably 1 amino acid, are substituted. The substituted amino acids may be substituted continuously and/or at interval.

Further, the amino acid sequence Ac-SRMKKWAKIIEKWRKWH-NH₂ has one and/or more amino acids added to the carboxyl terminal and/or amino terminal, and the antimicrobial activity of the extended amino acid sequence is not significantly reduced, or remains the same, or is significantly enhanced. Still further, 1-30 amino acids, preferably 1-20 amino acids, and more preferably 1-10 amino acids, are added. The added amino acids may be concentrated at the carboxyl terminal, or at the amino terminal, or at both the carboxyl and amino terminals.

Further, the amino acid sequence Ac-SRMKKWAKIIEKWRKWH-NH₂ has 1-5 amino acids added or deleted, preferably 1-3 amino acids added and/or deleted, and further preferably 1 amino acid added and/or deleted, and the added or deleted amino acids may be concentrated or dispersed at any position of the amino acid sequence, and the antimicrobial activity of the altered amino acid sequence is not significantly reduced, or remains the same, or is significantly enhanced.

Further, the spatial configuration of the amino acid sequence Ac-SRMKKWAKIIEKWRKWH-NH₂ or a mutant sequence thereof (the amino acid sequence after amino acid substitution, addition, or deletion as described above) is an α-helix configuration, a β-fold configuration, and/or an extended/randomly coiled configuration.

Furthermore, the nucleic acid molecule encodes an antimicrobial polypeptide, wherein the antimicrobial polypeptide comprises an amino acid sequence having 90%-100% homology with the amino acid sequence Ac-SRMKKWAKIIEKWRKWH-NH₂.

In a fifth aspect, the present disclosure provides a host cell, wherein the host cell is capable of expressing the antimicrobial polypeptide of the present disclosure.

In a sixth aspect, the present disclosure provides use of the antimicrobial polypeptide, wherein the use is to treat a bacterial infectious disease, a viral infectious disease, a chlamydial infectious disease, a fungal infectious disease, a mycoplasmal infectious disease, a rickettsial infectious disease, for example, rosacea, adolescent acne, atopic dermatitis, genital warts, vulval intraepithelial neoplasia, inflammatory responses, and inflammatory response syndrome.

### Brief Description of the Drawings

FIG. 1. Detection of antimicrobial activity of E.coli;
FIG. 2. Detection of antimicrobial activity of S. aureus;
FIG. 3. Toxicity experiment of AC-SH-17 on mammalian cells cultured in vitro;
FIG. 4. Detection of inflammatory response in cells; and
FIG. 5. Effect of Ac-SH-17 on the survival rate of LPS-induced sepsis mice.

### Detailed Description of the Embodiments

### Definition and general technology

Unless otherwise defined herein, the technical terms used in conjunction with the present disclosure shall have meanings commonly understood by those skilled in the art. In general, terminologies and technologies thereof used in conjunction with cell and tissue culture, molecular biology, immunology, microbiology, genetics as well as protein and nucleic acid chemistry and hybridization described herein are those well-known and commonly used in the art.

The term "polypeptide" encompasses both individual "polypeptide" and multiple "polypeptides", and refers to molecules composed of monomers (amino acids) linearly linked through amide bonds (also known as peptide bonds). The term "polypeptide" refers to any one or more chains containing two or more amino acids, and does not refer to products of a specific length. The polypeptides of the present disclosure may have a size of about 10-30 or 10-20 amino acids, and the polypeptides may have a limited spatial structure, but they do not necessarily need to have such a structure. In one embodiment of the present disclosure, the antimicrobial peptide is obtained by artificial chemical synthesis, and its carboxyl terminal is subjected to amidation treatment.

Vitellogenin (Vg) is a protein present in the blood of non-mammalian sexually mature animals of oviparous animals, belongs to the transport lipoprotein superfamily, and as a precursor of vitellin, is found in almost all oviparous animals, including fish, amphibians, reptiles, birds, and most invertebrates. Vitellogenin is generally synthesized exogenously and transported to the ovaries through the bloodstream.

Phosvitin (Pv) is formed in oocytes by the hydrolysis of Vg by protease D from the aspartic protease family. Pv not only has a conserved serine domain but also a high phosphorus content, making it one of the highest known natural proteins in terms of the phosphorus content. In chickens, Xenopus laevis, and bony fish, the Pv can contain up to 50% serine residues, which can be covalently linked to phosphates and ionically linked to calcium ions, enabling the Pv to transport phosphates and calcium ions.

The term "pharmaceutical composition" described herein is prepared by mixing the antimicrobial peptides of the present disclosure with a pharmaceutically acceptable carrier, and the carrier is in a form of a freeze-drying preparation or an aqueous solution. The pharmaceutically acceptable carrier is generally non-toxic to a subject in a dose and concentration used, and is prepared in a form of liquid, solid, aerosol or other oral administrations. In some aspects, the pharmaceutical composition provided in the present disclosure can be used for a method for treating a disease in a subject, wherein the method comprises administrating to a subject: antimicrobial peptides, antimicrobial peptides with site mutations, antimicrobial peptides with homologous sequences, peptide derivatives, or pharmaceutical compositions thereof.

The subjects of the pharmaceutical composition of the present disclosure include human and non-human animals, for example mammals such as mice, rats, guinea pigs, dogs, cats, rabbits, cows, horses, sheep, goats and pigs, birds, fish, reptiles, amphibians, etc. The term also includes cells and microorganisms. In one embodiment, the subject is selected from cells, and selected from mouse fibroblasts (L929), frozen cells, and human red blood cells (RBC).

The term "administration" that can be interchangeably used herein refers to the amount and mode of administration of the antimicrobial peptides, the antimicrobial peptides with site mutations, the antimicrobial peptides with homologous sequences, the peptide derivatives, or pharmaceutical compositions thereof in the present disclosure that are sufficient to improve one or more symptoms of the disease being treated.

Antimicrobial activity refers to the ability of antibiotics to inhibit or kill pathogenic microorganisms, and the lowest inhibitory concentration that can inhibit bacterial growth in the culture medium is the minimum inhibitory concentration (MIC). When using killing bacteria as the evaluation criterion, reducing the total number of viable bacteria by 99% or 99.5% or more is called the minimum bacterial concentration (MBC). In the embodiments of the present disclosure, the antimicrobial ability of the antimicrobial peptide Ac-SH-17 is tested using the minimum inhibitory concentration.

As used herein, the term "condition or disease" includes rosacea, adolescent acne, atopic dermatitis, genital warts, vulval intraepithelial neoplasia, inflammatory response syndrome, etc.

The materials of Gram negative bacteria and Gram positive bacteria used in the embodiments of the present disclosure include Gram negative bacteria Escherichia coli strain Escherichia coli 25922 (ATCC 25922, E. coli 25922) and Gram positive bacteria Staphylococcus aureus strain Staphylococcus aureus 25923 (ATCC 25923, S. aureus 25923).

The antimicrobial peptide is obtained by Gram positive bacterial through artificial chemical synthesis, its carboxyl terminal is subjected to amidation treatment, and the purity of the synthesized product is greater than 95%. After passing the quality inspection, the synthesized modified antimicrobial peptide is dissolved in 1% (v/v) 2,2,2-trifluoroethanol (TFE) and prepared as a mother liquor with a final concentration of 1 mg/mL, and after packaging, it is stored in a -20°C refrigerator.

The reagents used in the embodiments of the present disclosure are: LB nutrient broth, DMSO (Solarbio), DMEM nutrient solution (GIBCO), fetal bovine serum (Hyclone), 1.8 ml cryovial, 0.25% trypsin (GIBCO), PBS, DMEM nutrient solution containing 10% fetal bovine serum, DMSO, MTT, and pancreatin.

The required instruments for the embodiments of the present disclosure include: 4 ml sterile centrifuge tube, shaker (culture conditions 37°C, 150 r/min), water bath, centrifuge, inverted microscope, CO₂ incubator, ultra clean workbench, 96-well plate, water bath constant temperature oscillator, microplate reader, etc.

### Example 1 Synthesis of Ac-SH-17 Polypeptide

1. Amino resin with a substitution degree of about 0.5 mmol was taken and placed in a reaction tube, and DCM (15 ml/g) was added and shaken for 30 min.
2. Connecting the first amino acid: the solvent was removed by suction filtration through a sand core, a 3-fold molar excess of amino acid was added, DMF was added to dissolve, and then a 10 fold molar excess of DIEA was added, shaken for 60 min, and sealed with methanol.
3. Deprotection: DMF was removed, 20% piperidine DMF solution (15 ml/g) was added for 5 min, the piperidine DMF solution was removed and another 20% piperidine DMF solution (15 ml/g) was added for 15 min.
4. Detection: the piperidine solution was removed by suction filtration, more than ten pieces of resin was taken, washed three times with ethanol, detected with the added detection reagent, and heated at 105°C-110°C for 5 min, and a dark blue color indicated a positive reaction.
5. Washing: it was washed with DMF (10 ml/g) twice, with DCM (10 ml/g) twice, and with DMF (10 ml/g) twice.
6. Condensation: triple excess of protective amino acids and triple excess of HBTU were dissolved in as little DMF as possible and added to a reaction tube, and ten-fold excess of DIEA was immediately added to react for 30 min.

The operation was repeated, the amino acids in the Ac-SRMKKWAKIIEKWRKWH-NH₂ sequence was connected from right to left, and the last amino acid Fmoc protecting group was removed.

7. Cutting peptides from resin: a cutting solution (10 ml/g) was prepared from: TFA 95%; 1% water; EDT 2%; TIS 2%, with cutting time: 120 min.

8. Blowing dry and washing: the cutting solution was blown dry as much as possible with nitrogen, washed six times with ether, and then evaporated dry at room temperature.

9. Analysis and purification: a crude product was purified using high-performance liquid chromatography.

10. Freeze-drying: the target peptide solution was collected and placed in a freeze dryer for concentration, and then freeze-dried into powder.

### Example 2: Detection of Antimicrobial Activity of Modified Peptide Ac-SH-17

### 2.1 Qualitative analysis of the antimicrobial activity of modified peptides by liquid antimicrobial method

8 sterile 4 ml centrifuge tubes were prepared and numbered 1-8, 1.5 ml sterile nutrient broth was added to the sterile centrifuge tubes 2-8, then the prepared sample was taken and added to centrifuge tubes 1 and 2, and mixed well in the centrifuge tube 2, then 1.5 mL of the mixture was sucked out from the centrifuge tube 2 to centrifuge tube 3, and mixed well in the centrifuge tube 3, then 1.5 mL of the mixture was sucked out from the centrifuge tube 3 to centrifuge tube 4, ... and finally, 1.5 mL of liquid was drawn from centrifuge tube 7 and discarded. In this way, the centrifuge tubes 1-7 contained samples with multiplicatively reduced mass concentration, with centrifuge tube 8 as the negative control group and the centrifuge tube 1 as the positive control group. Finally, 1.5 mL of spare bacterial solution was added to each centrifuge tube. 3 parallel samples were made for each sample. 100 µl of the sample was immediately taken and placed in a microplate reader to measure its OD540, which was recorded as OD 0h. Then the sample was placed in a 37°C constant temperature incubator for cultivation, 100 µl of the sample was taken every 1 h, and the OD540 of each centrifuge tube was measured. Based on the changes in absorbance, the growth of bacteria could be determined, and the antimicrobial activity and minimum inhibitory concentration (MIC) of the polypeptide could be determined.

### 2.2 Quantitative analysis of antimicrobial activity of the modified peptides by turbidimetric method using the microplate reader

### 1. Preparation of bacterial solution

The bacterial solution was diluted with LB nutrient broth, and the concentration was adjusted to 104 CFU/mL.

### 2. Polypeptide renaturation

The polypeptide was dissolved in LB nutrient broth, and 1% trifluoroethanol was added for renaturing for 0.5 h.

### 3. Incubation and cultivation

8 sterile 4 ml centrifuge tubes were prepared and numbered 1-8, 1.5ml sterile nutrient broth was added to sterile centrifuge tubes 2-8, then the prepared sample was taken and added to centrifuge tubes 1 and 2, and mixed well in the centrifuge tube 2, then 1.5 mL of the mixture was sucked out from the centrifuge tube 2 to centrifuge tube 3, and mixed well in the centrifuge tube 3, then 1.5 mL of the mixture was sucked out from the centrifuge tube 3 to centrifuge tube 4, and finally, 1.5 mL of liquid was drawn from centrifuge tube 7 and discarded. In this way, the centrifuge tubes 1-7 contained samples with multiplicatively reduced mass concentration, with the centrifuge tube 8 as the negative control group and the centrifuge tube 1 as the positive control group. Finally, 1.5 mL of spare bacterial solution was added to each centrifuge tube. 3 parallel samples were made for each sample. 100 µl of the sample was immediately taken and placed in a microplate reader to measure its OD540, which was record as OD 0h. Then the sample was placed in a 37°C constant temperature incubator for cultivation, 100 µl of the sample was taken every 1 h, and the OD540 of each centrifuge tube was measured once.

### Example 3: Cytotoxicity and Hemolysis Analysis of Ac-SH-17

### 3.1 Cell toxicity experiment

1. Preparation of solution (5 mg/ml MTT): 15 mg of MTT was dissolved in 3 ml of PBS, filtered through a 0.22 µm filter membrane in an ultra-clean bench, and stored at 4°C in the dark (wrapped in tin foil);
2. 20 mg of each material was weighed, sterilized with UV for 24 h, and dissolved in a DMEM culture medium containing 10% fetal bovine serum to prepare a culture medium with five concentration gradients, i.e., 5 mg/ml, 2 mg/ml, 1 mg/ml, 0.5 mg/ml, and 0.25 mg/ml;
3. Inoculation of cells: L929 cells with vigorous growth were digested, the cell suspension was adjusted to 104 cells/ml, three 96 well cell drop plates were used, with 100 µl of cell suspension added to each well, and the cells were cultured in a CO₂ incubator for 24 h and adhered to the wall;
4. Sample addition
   Blank control group: PBS, cell-free
   Sample control group: Cells cultured in a culture medium containing materials
   Negative control group: Cells cultured in culture medium without materials
   Positive control group: culture medium containing 0.5% phenol

### 3-5 parallel samples were set per group

5. The original culture medium was discarded from the 96 well plate, and 100 µl/well of experimental group, blank group, normal control group were added, with 3-5 parallel samples in each group, and the three plates at 24 h, 48 h, and 72 h were cultivated in a CO₂ incubator;

### 6. Observation of cell morphology and measurement of OD value using MTT assay

Cell plates were taken out at 48 h, 72 h, and 96 h, and the cell morphology was observed under an inverted microscope, recorded, and photographed. Under low light conditions, the original solution was discarded from each well, 100 µl of culture medium and 20 µL of MTT were added, and incubated for 4-5 h in a CO₂ incubator at 37°C. The original solution was discarded, 150 µl of DMSO was added, and the mixture was shaken for 8 min. The OD value was measured at 490 nm using a microplate reader, and the relative cell growth rate (RGR) was calculated and evaluated for cytotoxicity RGR (%) = (Experimental group OD - Blank group OD)/(Normal group OD - Blank group OD) * 100%

### 3.2 Hemolytic test

Human red blood cells (RBC) were used to detect the hemolytic activity of Ac-SH-17. All subjects provided written informed consent. The obtained healthy human blood was placed in an EDTA anticoagulant tube. Red blood cells were collected by centrifugation and suspended in PBS to a concentration of 4% (v/v). An equal portion of 200 µl red blood cell suspension was mixed with 200 µl of Ac-SH-17 solution at different concentrations (12.5, 25, 50, 100, and 200 µg/ml). After incubation at 37°C, the mixture was centrifuged for 1 h, and the supernatant was collected and added to a 96-well plate. Red blood cells incubated with PBS, BSA solution (100 µg/ml), or 0.1% Triton X-100 solution were used as blank control, negative control, and positive control, respectively. The absorbance was measured at 540 nm using a microplate reader. Three parallel samples were set up for each experiment, and the experiment was repeated at least three times. The results were analyzed for significant differences by a two-way ANOVA method, with p<0.05 indicating significant differences.

**Table 3.1 Hemolysis analysis**

| **Hemolytic activity of polypeptide** | | |
|---|---|---|
| **Run** | **OD Value** | **Hemolysis Ratio (%)** |
| AC-SH-17 | 0.061±0.013 | 4.40% |
| Negative Control | 0.024±0.010 | / |
| Positive Control | 0.855±0.100 | / |

According to Table 3.1, compared with the positive control group, Ac-SH-17 does not have significant hemolytic effect. Therefore, it could be considered that Ac-SH-17 has no hemolytic activity on mammalian blood cells.

### Example 4: Anti-inflammatory Experiment of Antimicrobial Peptide

NO is a diffusible and membrane permeable small molecule obtained during the oxidation of L-arginine by NOS. NO was associated with many physiological reactions in organisms. NO exhibited cytotoxicity at high concentrations and had antimicrobial and insecticidal effects in mammalian cellular immunity. But at low concentrations, NO could act as a signal to positively and negatively regulate innate and acquired immunity. Thus, by detecting the concentration of NO released by cells, the inflammatory response of cells could be detected.

The RAW264.7 mouse macrophage cell line was used as the experimental material and cultured in a DMEM high glucose medium containing FBS (10%) and penicillin-streptomycin dual antibody (100 U/ml). The RAW264.7 mouse macrophage cell line was used for experiments when the cell state was stable, the shape was full, and the edges were transparent.

### 4.1 Cell treatment

1. The culture medium was removed from the cells and gently purged twice with PBS at room temperature to remove excess culture medium. After digestion with 0.25% trypsin for 3 min, the culture bottle was gently tapped to induce cell detachment, and then the culture medium was added to terminate digestion. The culture bottle was repeatedly blown and beaten to make the cells uniform.
2. A brand new sterile 6-well plate (Corning, item number 3516) was taken, 2 ml of cell suspension was added to each well, and the 6-well plate was gently shaken to ensure that the cells were evenly spread throughout the plate. Afterwards, the 6-well plate was incubated in a 37°C incubator for 4 h to ensure full cell adhesion and achieve a density of 1.5×10⁶ cells/ml.
3. After 4 h, the 6-well plate was removed, the culture medium was sucked out, the 6-well plate was washed twice with a PBS solution, and then Opti-MEM culture medium was added to continue culturing for 6 h to remove the effect of specific components in the serum on LPS.
4. The cells were randomly divided into 8 groups, i.e., (a) control group: a normal cultured cell group; (b) LPS treatment group: a cell group treated with LPS at a final concentration of 1 µg/ml; (c) BSA group: a cell group treated with 10 M BSA; (d) polypeptide group: a cell group treated with different concentrations of antimicrobial peptides; (e) LPS and BSA group: a cell group treated with LPS at a final concentration of 1 µg/ml and 10 M BSA; (f) LPS and polypeptide group: a cell group treated with LPS at a final concentration of 1 µg/ml and different concentrations of antimicrobial peptides.
5. 4 µL of LPS was added to the treatment group containing LPS to make the final concentration of LPS in the system 1 µg/ml, and PBS and BSA were added to the corresponding cell group which was returned to the incubator for 24 h to induce an inflammatory response. During this period, pay attention to observing the state of the cells. If the cells had antennae, they might undergo activation. At this time, the secretion of inflammatory factors by the cells was more complex and it was not suitable to continue the experiment.
6. The treated cells were removed from the incubator, and the supernatant was transferred to a new sterile test tube, and stored in a -20°C freezer for subsequent experiments.

4.2 In this experiment, NO was detected by the method provided in the total nitric oxide content determination kit, and improvements were made. The specific method was as follows:
1. Sample treatment: the supernatant was centrifuged with ultrafiltration tubes at room temperature at 14000 g, protein was removed after 5 min, and the lower clear liquid was retained for subsequent measurements.
2. Diluting standard solution: 1 ml of sterilized ddH₂O was added to KNO₂ dry powder to prepare a 10 mM solution, mixed evenly, and diluted to standard solutions of 2, 5, 10, 20, and 50 µmol/L for later use.
3. Preparation of reagents:
   A. About 1 ml of sterilized ddH₂O was added to 5 mg of NADPH, inverted and mixed until dissolved, then diluted to 3 ml with ddH₂O to prepare 2 mM NADPH. Except for the immediately used portion, the remaining NADPH solution must be immediately packaged and stored at -80°C;
   B. FAD, LDH, and Nitrateuctase were separately packaged and stored at -80°C for future use;
   C. Before use, the Nitrateuctase was taken out, and the remaining reagents were placed on an ice bath after dissolution. Griess Reagent I and Griess Reagent II needed to be equilibrated to room temperature before use;
4. Detection of NO:
   A. 30 µl of the sample or standard were taken and placed in a 96-well plate, with 3 replicates per sample.
   B. 2.5 µl of NADPH, 5 µl of FAD, and 2.5 µl of Nitrateuctase were added to the sample separately, and mixed thoroughly and evenly, and be careful to avoid capillary suction during the sample addition process. The mixture was incubated at 37°C for 15 min, or at room temperature (20°C-30°C) for 40 min.
   C. 5 µl of LDA Buffer and 5 µl of LDH were added separately to the sample and mixed thoroughly and evenly. The mixture was incubated at 37°C for 5 min, or at room temperature (20°C-30°C) for 20 min.
   D. 25 µl of Griess Reagent I and Griess Reagent II were added in sequence, mixed well, and incubated at room temperature (20°C-30°C) for 10 min. Then the absorbance at 540 nm was measured and the reading was recorded.
5. A standard curve was created, and the concentration of nitric oxide in the sample was calculated.

### Example 5 Antisepsis Experiment of Antimicrobial Peptide

Sepsis refers to a systemic inflammatory response syndrome caused by infectious factors. A rat model of intestinal sepsis was established by injecting rat fecal homogenate into the abdominal cavity of model rats, followed by intraperitoneal injection of antimicrobial peptides to observe the survival rate of mice within 10-48 h, in order to evaluate the therapeutic effect of antimicrobial peptides on sepsis in mice.

### 5.1 Grouping of experimental animals.

60 SD rats were randomly divided into 6 groups by a random number table method, i.e., model group 1, model group 2, model group 3, model group 4, model group 5, and blank group, with 10 rats in each group. These 6 groups of rats were weighed and numbered in sequence.

### 5.2 Reproduction of endotoxemia rat model.

### 5.3 Preparation of fecal homogenate supernatant.

After 1 week of adaptive feeding, fresh formed feces were collected from these 60 SD rats. After weighing the rat feces, an appropriate amount of physiological saline was added to it, and then the mixture was placed in a homogenizer for thorough grinding to prepare a rat feces homogenate at the concentration of 10%. The rat fecal homogenate was centrifuged at 3000 rpm for 5 min to obtain the supernatant, and then a syringe was used to suck out an appropriate amount of the supernatant for later use.

### 5.4 Preparation of rat model.

Before modeling, rats in model groups 1-5 were fasted for 12 hours, and then a 10% concentration of rat feces homogenate supernatant was injected intraperitoneally at a dose of 1 ml/200 g. After injection, the signs and manifestations of these 5 groups of rats were observed. If these 5 groups of rats showed signs such as curling up, lack of movement, mental fatigue, hair loss, chills, and obvious bloodstains around the nose, it indicated successful modeling. The successfully modeled rats would be used as the research subjects for this study.

The model rats were randomly divided into two groups, i.e., a model group and an administration group. After successful modeling, the administration group was intraperitoneally injected with 0.5 ml of antimicrobial peptide (80 µM), and each group was given the same amount of water and feed, and housed in separate cages. The activity and feeding habits of all mice were observed.

### Example 6: Cell Lines and Culture Thereof

This study used mouse epithelioid fibroblast L929 cells as experimental materials and cultured them in a DMEM medium containing 10% FBS. The culture environment was set as 37°C and 4% CO₂.

### 6.1 Cell recovery

1. The cryopreservation tube was taken out from the liquid nitrogen tank and quickly placed in a 37°C water bath (1-2 min for dissolution);
2. The culture medium (1 ml frozen cells + 4 ml fresh culture medium) was added, transferred to a culture bottle, and incubated at a constant temperature;
3. Small black dots suspended were observed under an inverted microscope.

### 6.2 Cell passage

1. Culture medium (containing suspended cells): the culture medium was pipetted to a sterile centrifuge tube;
2. Wall adhesion part: cells were washed with 1 ml of PBS, PBS was sucked out, and the cells were collected in a centrifuge tube containing culture medium in step 1;
3. 1 ml of trypsin was added to the culture bottle and placed in the incubator for digestion;
4. The digestion time varied depending on the characteristics of the cells; under the microscope, the cells visibly shrank and became round; when the cells in the lateral culture bottle could slide, digestion could be terminated, and the culture bottle could not be tapped throughout the process;
5. The digestion was terminated with 3ml of serum-containing culture medium, the digested cell suspension was dispersed and collected in the centrifuge tube of step 1;
6. The centrifuge tube was centrifuged at 1200 rpm (about 250 g) for 3 min, the supernatant was discarded after centrifugation, new culture medium was added and resuspended, divided into bottles according to the actual situation, and placed in the incubator for cultivation.

### 6.3 Cell cryopreservation

1. UV sterilization was performed on the ultra-clean workbench for 30 min, ventilation was performed for 10 min, the culture medium, trypsin, PBS were preheated at 37°C, and instruments and cryovials were sterilized at high temperature and high pressure.

### 2. Washing off the cells

The liquid was sucked out from the cell culture bottle, washed once with 3 ml of PBS, sucked out, and digested with 3ml of 0.25% trypsin for 3 min; the digestion was terminated with 2 ml of digestion solution; the cells washed off were repeatedly blown and beaten and centrifuged at 1000 g for 3 min; the precipitated cells were suspended in 1.2 ml of cryopreservation solution, placed in a cryopreservation tube, sealed, refrigerated at 4°C for 30 min, refrigerated at 20°C for 1 h, refrigerated overnight at -80°C and stored in a liquid nitrogen tank.

### Example 7: Experimental Results

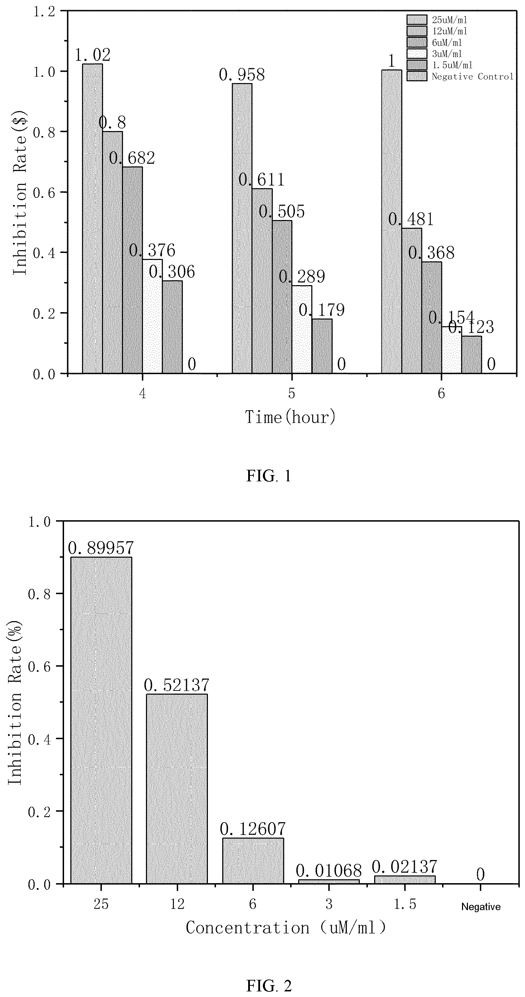

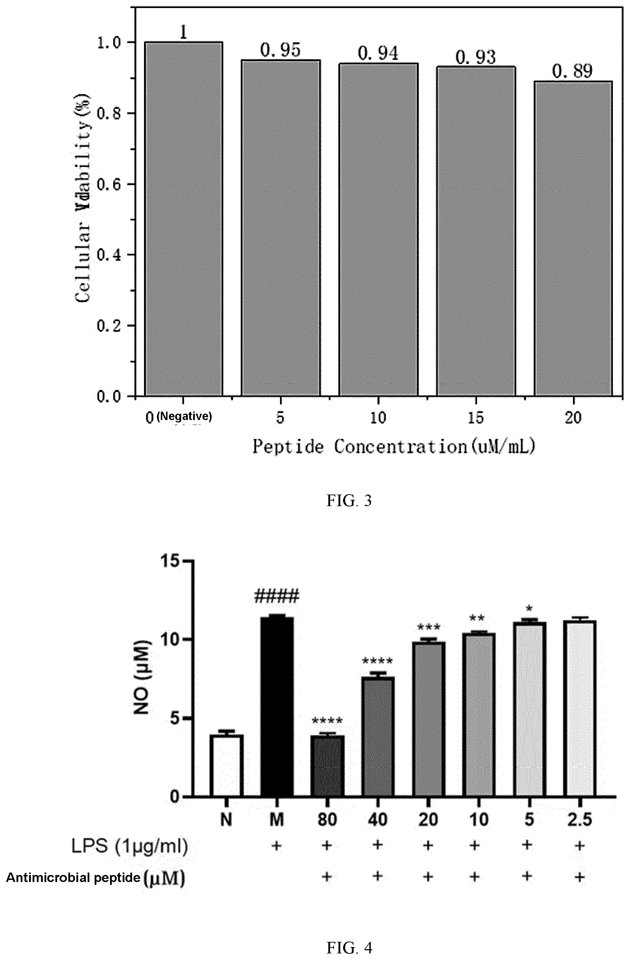

As shown in FIGs. 1 and 2, the modified antimicrobial peptide Ac-SH-17 exhibited antimicrobial activity against two drug sensitive strains, which could be seen from the results in FIG. 3, and the polypeptide AC-SH-17 did not show significant toxic or damaging effects at 20 µM. Therefore, it could be inferred that AC-SH-17 did not have significant toxicity to mammalian normal tissue cells cultured in vitro.

In FIG. 4, after inducing cell inflammatory response with 1 µg/ml LPS stimulation, a release concentration of NO reached 12 µM (M), and later, different concentrations of antimicrobial peptides were added, and it was found that when the antimicrobial peptide concentration reached 80 µM, it significantly inhibited the release of NO, and a release concentration of NO was substantially the same as that of the blank control. Therefore, it could be concluded that the antimicrobial peptides could effectively inhibit the inflammatory response of cells under certain concentration conditions.

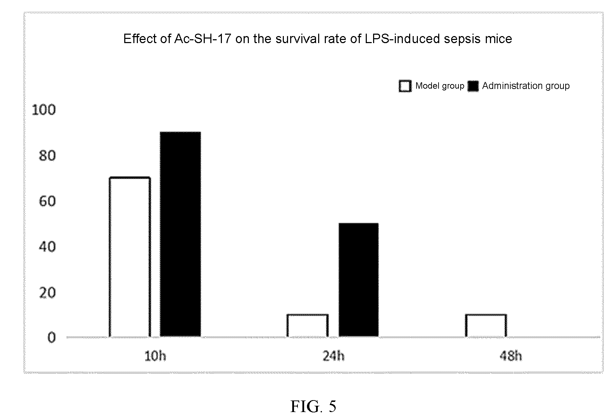

After 48 h of feeding mice under the same conditions, it was found in FIG. 5 that the administration group significantly improved the survival rate of mice with sepsis within 24 h compared to the model group. Therefore, it could be considered that the antimicrobial peptide Ac-SH-17 has a good therapeutic inhibitory effect on sepsis.

## Claims

1. A novel antimicrobial polypeptide, comprising the amino acid sequence Ac-SRMKKWAKIIEKWRKWH-NH₂.

2. The novel antimicrobial polypeptide according to claim 1, wherein the amino acid sequence Ac-SRMKKWAKIIEKWRKWH-NH₂ can have one and/or more amino acids substituted, and the antimicrobial activity of the substituted amino acid sequence is not significantly reduced, or remains the same, or is significantly enhanced.

3. The novel antimicrobial polypeptide according to claim 1, wherein the amino acid sequence Ac-SRMKKWAKIIEKWRKWH-NH₂ has one and/or more amino acids added to the carboxyl terminal and/or amino terminal, and the antimicrobial activity of the extended amino acid sequence is not significantly reduced, or remains the same, or is significantly enhanced.

4. The novel antimicrobial polypeptide according to claim 1, wherein the amino acid sequence Ac-SRMKKWAKIIEKWRKWH-NH₂ has 1-5 amino acids added or deleted, and the added or deleted amino acids can be concentrated or dispersed at any position of the amino acid sequence, and the antimicrobial activity of the altered amino acid sequence is not significantly reduced, or remains the same, or is significantly enhanced.

5. A novel antimicrobial polypeptide, comprising an amino acid sequence having 90%-100% homology with the amino acid sequence Ac-SRMKKWAKIIEKWRKWH-NH₂.

6. An antimicrobial pharmaceutical composition, comprising the antimicrobial polypeptide according to any one or more of claims 1-5, and a pharmaceutically acceptable excipient.

7. An antimicrobial pharmaceutical composition, further comprising an additional active ingredient different from the antimicrobial polypeptide according to any one or more of claims 1-5, wherein the additional active ingredient can be an antibiotic, an immunomodulator, a mineral, a trace element and/or a vitamin.

8. A nucleic acid molecule, wherein the nucleic acid molecule encodes an antimicrobial polypeptide, and the antimicrobial polypeptide comprises the amino acid sequence Ac-SRMKKWAKIIEKWRKWH-NH₂.

9. A host cell, wherein the host cell is capable of expressing the antimicrobial polypeptide according to any one or more of claims 1-5.

10. Use of the antimicrobial polypeptide according to any one of claims 1-5 in the preparation of a drug for the treatment of a microbial infectious disease, wherein the microbial infectious disease includes a bacterial infectious disease, a viral infectious disease, a chlamydial infectious disease, a fungal infectious disease, a mycoplasmal infectious disease and/or a rickettsial infectious disease.
